# EUROPÄISCHE PATENTSCHRIFT

(11) **EP 0 624 104 B1**
(45) Veröffentlichungstag und Bekanntmachung des Hinweises auf die Patenterteilung: **30.07.1997**
(21) Anmeldenummer: 93922943.1
(22) Anmeldetag: 13.10.1993
(51) Int. Cl.: A61M 25/06, A61B 17/34

(54) **KATHETERISIERUNGSBESTECK**
CATHETERISATION SET
ENSEMBLE DE CATHETERISME

(30) Priorität: 24.11.1992 DE 9215927 U
(43) Veröffentlichungstag der Anmeldung: 17.11.1994
(60) Teilanmeldung: 96118183.1
(73) Patentinhaber: B. BRAUN MELSUNGEN AG, 34212 Melsungen (DE)
(72) Erfinder: JESCH, Franz, D-82152 Krailling (DE)
(74) Vertreter: Selting, Günther, Dipl.-Ing.
(86) Internationale Anmeldenummer: EP9302813
(87) Internationale Veröffentlichungsnummer: WO9412233

(56) Entgegenhaltungen:
- EP-A- 0 129 745
- EP-A- 0 161 636
- EP-A- 0 352 928
- EP-A- 0 411 605
- WO-A-88/07388
- WO-A-92/18193
- WO-A-93/11812
- DE-A- 2 052 364
- DE-U- 8 914 941
- DE-U- 8 915 299
- FR-A- 2 368 968
- US-A- 3 680 562
- US-A- 4 191 186

## Beschreibung

Die Erfindung bezieht sich auf ein Katheterisierungsbesteck für die Katheterverlegung in einem Blutgefäß, bestehend aus einer Punktionskanüle mit einem Kanülenansatz, der eine Griffvorrichtung aufweist, einem die Punktionskanüle umgebenden Katheter mit einem Katheteransatz, und einem Führungsdraht, der in dem Lumen der Punktionskanüle axial verschiebbar ist.

Zur Messung von Drücken in Gefäßen (arterieller Blutdruck) sowie zur Verabreichung von Infusionen und Blutabnahmen müssen Arterien und zentrale Venen punktiert und sogenannte Verweilkanülen plaziert werden, die als kurze Katheter mit einem Katheteransatz gestaltet sind, an den eine Flüssigkeitsübertragungsvorrichtung anschließbar ist. In dem Katheter steckt eine Punktionskanüle, deren angeschärfte Spitze über das vordere Ende des Katheters etwas vorsteht. Ein solches Besteck ist bekannt aus US-A-3 312 220. Damit bei der Punktion der Übergang von der Punktionskanüle auf den dickeren Katheter allmählich erfolgt, ist die Spitze des Katheters außen nach vorne abgeschrägt. Der stufenlose Anschluß des Katheters an den Außenumfang der Punktionskanüle vermag jedoch nicht die Nachteile der Punktionslochaufweitung und der Erschwerung der Durchdringung der Haut und Gefäßwand zu vermeiden, die die Präzision der Punktion verringern. Letzteres wird dadurch verstärkt, daß bei diesem Besteck eine Griffvorrichtung fehlt, so daß es direkt am zylindrischen Kanülenansatz angefaßt werden muß und hier nur unsicher festgehalten werden kann.

Häufig ist es günstig, zur Plazierung des Katheters einen Führungsdraht zu verwenden. Dieser ist länger als der Katheter. Zur Verhinderung von Verletzungsgefahren haben Führungsdrähte eine geschmeidige Spitze. Der Vorteil des Führungsdrahtes besteht darin, daß er sich - da er steifer ist als der Katheter - zielsicherer als dieser im Gefäß vorschieben läßt und daß er den über ihn vorgeschobenen Katheter versteift und geführt zum Zielort bringt. Diese Praxis der Katheterverlegung wird "Seldinger-Technik" genannt, und sie wird sowohl bei kurzen als auch bei langen Kathetern angewendet. Es wird dabei im allgemeinen zunächst das Blutgefäß mit dem aus Punktionskanüle und aufgeschobenem Kurzkatheter bestehenden Besteck oder ausschließlich mit einer Punktionskanüle punktiert. Sodann wird durch die plazierte Punktionskanüle der Führungsdraht in das Gefäß eingeführt und es wird anschließend über den Führungsdraht der Katheter in das Gefäß vorgeschoben. Der Führungsdraht wird dann herausgezogen und verworfen. Die getrennt verwendeten Einzelteile sind für den Anwender unbequem, weil das Einfädeln bzw. Auffädeln der Teile zeitaufwendig ist und besondere Aufmerksamkeit verlangt.

Zur Vermeidung der Nachteile der Handhabung von Einzelteilen wurden diese zu einem Katheterisierungsbesteck zusammengebaut, das als Einheit handhabbar ist. Ein derartiges Katheterisierungsbesteck ist in EP-A-00 93 164 beschrieben. Dabei ist hinten (proximal) an den Kanülenansatz der mit dem Kurzkatheter zusammengesteckten Punktionskanüle ein langes Rohrteil angesetzt, das den Führungsdraht enthält, der mit Hilfe eines durch einen Schlitz in das Innere des Rohrteils ragenden Schiebers axial verschiebbar ist derart, daß sein vorderes Ende aus der Punktionskanüle Vortritt. Dieses Katheterisierungsbesteck läßt sich infolge seiner beträchtlichen Länge und des Fehlens einer Griffvorrichtung schlecht handhaben, was sich nachteilig auf die Punktionssicherheit auswirkt.

Die Punktion erfolgt mit zurückgezogenem Führungsdraht, damit bei Punktionserfolg Blut in der Punktionskanüle zurückströmt und in den transparenten Kanülenansatz gelangt. Da das Besteck jedoch an diesem Kanülenansatz gehalten wird, verdecken die Finger ihn und der Blutaustritt wird nicht oder erst spät im Kanülenansatz entdeckt. Auch bei diesem Besteck ist ungünstig, daß bei der Gefäßpunktion nicht nur die Punktionskanüle, sondern auch der diese umgebende Katheter das Punktionsloch durchdringen muß. Der Hautdurchtritt erfolgt aufgrund des Hautwiderstandes ruckartig und die genaue Plazierung der Punktionskanülenspitze im Gefäß wird erschwert bzw. häufig unmöglich.

Ein Katheterisierungsbesteck ist in EP-A-0446 804 beschrieben, bei dem die Handlichkeit durch eine Griffvorrichtung verbessert wird, die Griffplatten aufweist. Je eine Griffplatte ist an dem Katheteransatz und dem Kanülenansatz so angebracht, daß ihre Ebenen zur Längsachse des Bestecks quergerichtet sind und das vordere Ende des jeweiligen Ansatzes durchqueren. Zur Spitze der Punktionskanüle sind die Griffplatten weit nach hinten versetzt, so daß bei der Punktion ein langer Hebelarm wirksam ist, der eine präzise Punktion von Haut und Gefäßwand trotz des Vorteils besserer Erfaßbarkeit des Bestecks behindert.

Ein Katheterisierungsbesteck, von dem der Oberbegriff des Patentanspruchs 1 ausgeht, ist bekannt aus WO 88/07388. Dieses Besteck weist eine Punktionskanüle mit Kanülenansatz und einen auf der Punktionskanüle sitzenden Katheter mit Katheteransatz auf. An dem Kanülenansatz ist eine Griffvorrichtung vorgesehen, welche einen parallel zur Kanüle nach vorne (distal) vorstehenden Arm aufweist, der als längslaufendes Griffstück ausgebildet ist. An dem Katheteransatz sind zwei Grifflügel schwenkbar angebracht, die beim Ergreifen seitlich gegen das Griffstück des Kanülenansatzes gedrückt werden, um Kanüle und Katheter gleichzeitig festhalten zu können.

Aus FR-A-2 368 968 ist ebenfalls ein Katheterisierungsbesteck bekannt, bei dem der Kanülenansatz zwei gabelförmig nach vorne abstehende Griffstücke aufweist, die den auf die Kanüle aufgeschobenen Katheteransatz umgreifen. Auch hierbei soll mit derjenigen Hand, die die Kanüle führt, zugleich der Katheteransatz an dem Kanülenansatz festgehalten werden.

Der Erfindung liegt die Aufgabe zugrunde, ein Katheterisierungsbesteck zu schaffen, das eine exakte Führung der Punktionskanüle an der Punktionsstelle ermöglicht.

Die Lösung dieser Aufgabe erfolgt erfindungsgemäß mit den Merkmalen des Patentanspruchs 1.

Das in Richtung der Einstechspitze der Punktionskanüle nach vorne verlagerte Griffstück der Griffvorrichtung bewirkt eine direkte Einleitung der Vorschubkraft in die Punktionsnadelspitze beim Durchdringen von Haut und Gefäßwand mit der Folge einer präzisen Führung der Punktionskanüle zur Durchführung einer exakten Punktion. Das Punktionsbesteck läßt sich gut anfassen und handhaben, weil der Hebelarm zwischen Griffstück und Punktionskanülenspitze kurz ist und der Anwender sich mit der punktierenden Hand in der unmittelbaren Nähe des Punktionsortes abstützen kann, was die Zielsicherheit erheblich erhöht. Die Entfernung zwischen Griffstück und Punktionskanülenspitze kann in Anpassung an den Bestimmungszweck des Katheterisierungsbestecks weitgehend optimal gewählt werden. Vorteilhafterweise befindet sich zumindest das Vorderteil des Griffstücks etwa in der Längsmitte der Punktionskanüle. Die Länge des auf die Punktionskanüle aufgeschobenen Katheters ist von der Anordnung des Griffstückes unabhängig. Der Vorschub des aus Punktionskanüle, Katheter und Führungsdraht zusammengesetzten Katheterisierungsbestecks mit Hilfe des nach vorne versetzten Griffstücks erfolgt in jeder Phase der Anwendung präzise, so daß sowohl das Punktieren als auch das Einführen des Führungsdrahtes und das Nachführen des Katheters über den Führungsdraht ablenkungsfrei geschieht. Der Vorgang der Punktion und der Katheterverlegung wird damit für den Patienten sicherer und für den Anwender einfacher.

Das Griffstück ist an einem Arm vorgesehen, der ausgehend vom Kanülenansatz parallel neben der Punktionskanüle verläuft. Arm, Griffstuck und Kanülenansatz können einstückig aus Kunststoff hergestellt sein. Vorzugsweise ist der Arm flach ausgebildet und mit Randverstärkungen versehen, wobei die Ebene der Armfläche die Längsachse des Bestecks durchquert. Es wird hierdurch ein Federn des Armes vermieden und eine gute Kraftübertragung vom Griffstück über den Kanülenansatz auf die Kanülenspitze erreicht.

In zweckmäßiger Ausgestaltung der Erfindung weist das Griffstück zwei in gleicher Flucht zueinander parallele Griffplatten auf, deren Ebenen zur Längsachse des Bestecks quergerichtet sind. Beide Griffplatten werden zwischen Daumen und Zeigefinger einer Hand erfaßt und verbessern die Erfaßbarkeit und Kraftübertragung auf die Punktionskanülenspitze zusätzlich.

Mit dem erfindungsgemäßen Katheterisierungsbesteck erfolgt die Kathetereinführung nach zwei Methoden:
a) über die noch in der Arterie steckende Punktionskanüle zur besseren Führung durch Haut und Stichkanal, wobei der Führungsdraht die Führung im Lumen der Arterie übernimmt,
b) über den Führungsdraht bei vorher zurückgezogener Punktionskanüle.
Auf beide Methoden wirkt sich die gute Festhaltungsmöglichkeit des Bestecks mit Hilfe der erfindungsgemäßen Griffstückanordnung günstig aus.

Die Erfindung betrifft ferner ein Punktionsinstrument, das aus einer Stahlnadel besteht, die an einem Ende einen Ansatz trägt, der eine Griffvorrichtung aufweist, gemäß Anspruch 3. Die Stahlnadel kann eine hohle Punktionskanüle oder ein massiver Trokar sein, die beide am distalen Ende eine angeschärfte Einstichspitze aufweisen - in beiden Fällen ist es wichtig, daß sie sich so festhalten und führen lassen, daß die Einstichspitze sich präzise plazieren läßt. Dies wird durch die Anbringung des Griffstücks entfernt vom Ansatz und Verlagerung gegen die Einstichspitze erreicht.

In der Zeichnung ist ein Ausführungsbeispiel der Erfindung schematisch dargestellt. Es zeigen:

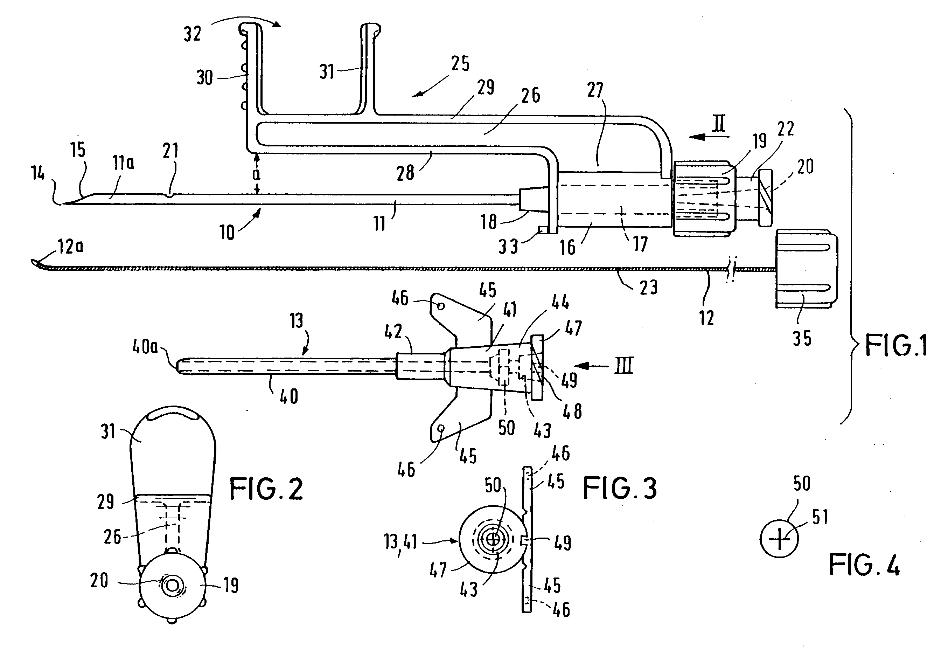

- Fig. 1: drei Bestandteile eines Katheterisierungsbestecks, die im Anwendungszustand koaxial zusammengesteckt sind,
- Fig. 2: eine Ansicht in Richtung des Pfeils II in Fig. 1,
- Fig. 3: eine Ansicht in Richtung des Pfeils III in Fig. 1,
- Fig. 4: eine Draufsicht auf eine Ventilscheibe für den Katheteransatz,
- Fig. 5: eine Draufsicht auf das anwendungsbereite Katheterisierungsbesteck,
- Fig. 6: das vordere Ende des Katheterisierungsbestecks in vorgeschrittenem Anwendungsstadium, und
- Fig. 7: den im Gefäß verlegten und auf der Haut festgenähten Katheter kurz vor der Öffnung des Ventils im Katheteransatz durch einen Offenhalter an einer Anschlußvorrichtung.

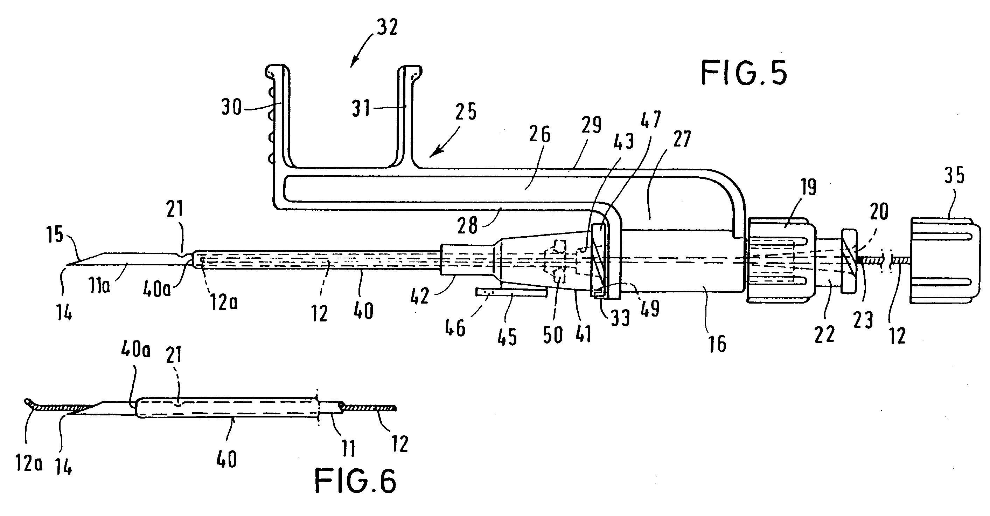

Ein Katheterisierungsbesteck, das sich besonders zur Katheterisierung von Arterien eignet, besteht im wesentlichen aus drei Teilen, nämlich einer Punktionskanüle 10, einem Führungsdraht 12 und einem Katheter 13, die sich - wie in Fig. 5 gezeigt - koaxial zusammenstecken lassen. Die Punktionskanüle 10 weist eine hohle Stahlnadel 11 auf, deren vorderes (distales) Ende 15 spitz und scharf angeschliffen ist und an deren hinterem (proximalen) Ende ein Kanülenansatz 16 befestigt ist, der einen durchgehenden Kanal 17 enthält. Ein Außenkonus 18 am Übergang zwischen der Stahlnadel 11 und dem Kanülenansatz 16 dient als Steckelement zur Verbindung mit einem Innenkonus eines Katheteransatzes des Katheters 13. Eine Blutrückhaltekappe 19 mit einem koaxialen engen Durchlaß 20 zur Führung des Führungsdrahtes 12 schließt den Kanülenansatz 16 am hinteren Ende ab. Die Blutrückhaltekappe 19 trägt am hinteren Ende einen koaxialen Kupplungsstutzen 22 mit einem Verriegelungselement für einen Anschluß. In einem Abstand von etwa 18 mm hinter der Spitze 14 der Stahlnadel 11 ist in ihrer Wand ein kleines Loch 21 ausgebildet, das gebohrt, geschliffen oder gestanzt sein kann und das von außen bis in das Lumen der Stahlnadel 11 durchgeht.

Mit dem Kanülenansatz 16 der Punktionskanüle 10 ist eine Griffvorrichtung 25 verbunden. Vorzugsweise sind der Kanülenansatz 16 und die Griffvorrichtung 25 aus Kunststoff gefertigt und einstückig hergestellt. Die Griffvorrichtung 25 weist einen Arm 26 auf, der parallel zur Stahlnadel 11 mit Abstand neben dieser verläuft. Das hintere (proximale) Ende des Armes 26 schließt sich über einen rechtwinklig abgehenden Schenkel 27 an einen rohrförmigen Hauptkörper des Kanülenansatzes 16 an. Vorzugsweise ist die Breite des Schenkels 27 im wesentlichen gleich der Länge des Hauptkörpers. Seine Erstreckung in Querrichtung bestimmt den Abstand a zwischen der Stahlnadel 11 und dem ihr nächstliegenden Rand 28 des Armes 26. Der Hauptbereich des Armes 26 ist plattenartig flach während seine beiden Längsränder 28 und 29 zur Stabilisierung wulstartig verstärkt sind. An dem vorderen (distalen) Ende des Armes 26 sind zwei Griffplatten 30, 31 angeformt, die in gleicher Flucht von dem Längsrand 29 rechtwinklig zur Seite abstehen. Die beiden Griffplatten 30, 31 bilden ein Griffstück 32, das zwischen Daumen und Zeigefinger erfaßt werden kann. Die Länge des Armes 26 und die Anbringung des Griffstückes 32 an diesem sind so gewählt, daß das Griffstück 32 sich im Mittelbereich der Stahlnadel 11 befindet. Ein bevorzugter Abstand zwischen der Spitze 14 und der Griffplatte 30 beträgt etwa 45 mm.

Durch den Kanülenansatz 16 und die Stahlnadel 11 ist der Führungsdraht 12 hindurchführbar, dessen Durchmesser dem Lumendurchmesser angepaßt ist und der an seinem proximalen Ende ein Kopfstück 35 trägt, das als Verschlußkappe mit Innengewinde ausgebildet und mit einem Gewindeabschnitt des Kupplungsstutzens 22 verriegelbar ist. Der Führungsdraht 12 kann als eng gewickelter Stahldraht mit flexibler Spitze 12a gestaltet sein. Das Katheterisierungsbesteck ist jedoch auch mit einem Führungsdraht funktionsfähig, dessen hinteres Ende als starrer Draht ausgeführt ist und der nur im vorderen Teil als schraubenförmig gewikkelter flexibler Strangkörper ausgebildet ist. Wenn der Führungsdraht 12 bis zum Anschlag des Kopfstückes 35 gegen die Blutrückhaltekappe 19 in die Punktionskanüle 10 eingeschoben und durch eine Drehbewegung mit dem Kupplungsstutzen 22 der Blutrückhaltekappe 19 verriegelt ist, überragt sein vorderes Ende die Spitze 14 der Stahlnadel 11 um ein zur Durchführung der Führungsfunktion im jeweiligen Anwendungsfalle benötigtes Stück, das etwa 20 mm bis 50 mm lang sein kann.

Der Katheter 13 ist ein Kurzkatheter aus flexiblem Kunststoff, der 45 mm bis 50 mm lang sein kann und aus einem Kapillar 40 besteht, an dessen hinterem Ende ein Katheteransatz 41 befestigt ist. Der Katheteransatz 41 ist ein Rohrstück, das am vorderen (distalen) Ende einen flexiblen Knickschutz 42 für das Kapillar 40 aufweist und am hinteren (proximalen) Ende einen Innenkonus 43 zur Aufnahme des Außenkonus 18 der Punktionskanüle 10 sowie eine Kammer 44 zur Unterbringung einer Ventilvorrichtung enthält. An der Außenseite des Katheteransatzes 41 sind zwei nach entgegengesetzten Seiten gerichtete Fixierflügel 45 angebracht, die Löcher 46 zum Festnähen auf der Haut des Patienten aufweisen.

Die hintere Öffnung des Katheteransatzes 41 ist von einem Ringflansch 47 umgeben, der Kupplungselemente 48, z. B. Gewindeteile, zur Verbindung mit einem Konnektor aufweist, der weiterführende Leitungen an den Katheter 13 anschließt. In dem Ringflansch 47 ist eine radiale Kerbe 49 ausgebildet, in die ein axialer Zapfen 33 des Kanülenansatzes 16 als Drehsicherung eingreift. In der Kammer 44 sitzt eine Elastomerscheibe 50, die von einem Schlitz oder Kreuzschlitz 51 durchsetzt ist, dessen Lippen dicht aneinander liegen und den Kanal des Katheters 13 nach hinten absperren.

Für den Transport in steriler Verpackung ist der Katheter 13 auf die Punktionskanüle 10 aufgesteckt und durch Zusammengriff des Außenkonus 18 mit dem Innenkonus 43 an dieser festgelegt. In diesem Zusammenbau-Zustand überragt die Stahlkanüle 11 mit einem Abschnitt 11a die Spitze 40a des Kapillars 40 um vorzugsweise 20 mm und das Loch 21 liegt frei vor der Kapillarspitze 40a (Fig. 5). Der Führungsdraht 12 ist durch die Punktionskanüle 10 hindurchgeschoben und ragt aus dem Schliffende 15 der Stahlkanüle 11 ein Stück vor. Eine nicht gezeichnete Schutzkappe deckt die in den Patienten einzuführenden Punktionselemente ab.

Zur Punktion wird der Führungsdraht 12 zurückgezogen bis eine auf dem Führungsdraht 12 angebrachte Markierung 23 mit der Hinterkante der Blutrückhaltekappe 19 längengleich übereinstimmt und seine Spitze 12a hinter dem Loch 21 der Stahlnadel 11 liegt (Fig. 5). Das Katheterisierungsbesteck wird an dem abgewinkelten Griffstück 32 erfaßt und es wird die Stahlnadel 11 ausschließlich mit ihrem von dem Kapillar 40 freien blanken Teil 11a in ein Blutgefäß eingestochen. Das Loch 21 bleibt außerhalb der Einstichstelle sichtbar. Die erfolgreiche Punktion ist durch Blutaustritt aus dem Loch 21 unverzüglich erkennbar. Sodann wird der Führungsdraht 12 in das Gefäß vorgeschoben und sein Kopfstück 35 mit der am Kanülenansatz 16 befestigten Blutrückhaltekappe 19 verriegelt. Der Vorschub des Führungsdrahtes 12 verschließt das Loch 21 und beendet den Blutaustritt. Auch ein Blutrückstrom wird von dem Führungsdraht 12 weitestgehend unterbunden, da dieser infolge seines dem Lumen der Stahlnadel 11 angepaßten Durchmessers als Lumenverschluß wirksam ist. Das Kapillar 40 kann über die aus dem Punktionsloch etwas zurückgezogene Stahlnadel 11 und den Führungsdraht 12 in das Gefäß geschoben werden. Anschließend werden die Stahlnadel 11 mit dem innenliegenden Führungsdraht 12 in toto aus dem Katheter 13 herausgezogen. Der Kreuzschlitz 51 der Elastomerscheibe 50 im Katheteransatz 41, der sich zum Durchlaß der Stahlnadel 11 ohne weiteres geöffnet und mit seinen vier Lappen an den Umfang der Stahlnadel 11 abdichtend angelegt hat, schließt sich nach Herausziehen von Stahlnadel 11 und Führungsdraht 12 abdichtend, so daß es nicht zum Blutrückstrom aus dem Katheter 13 kommt. Der Katheter 13 wird mittels der Fixierflügel 45 auf der Haut des Patienten festgenäht, so daß die Spitze 40a des Kapillars 40 in der korrekten Position innerhalb des Gefäßes festgelegt ist.

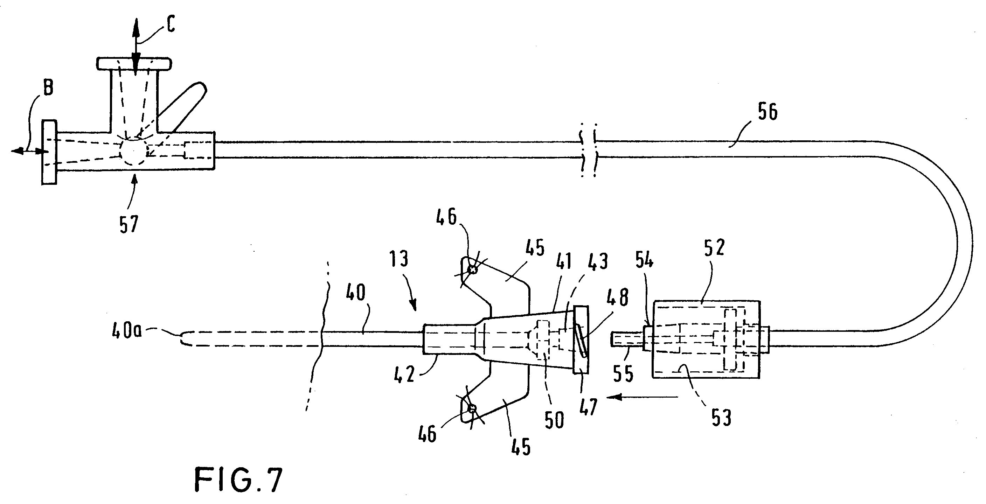

Zum Anschluß eines Übertragungsgerätes an den Katheter 13 dient eine flexible Schlauchleitung 56, deren eines Ende ein Gehäuse eines Dreiwegehahns 57 mit Ein- bzw. Ausgängen B und C trägt (Fig. 7). Am anderen Ende der Schlauchleitung 56 befindet sich ein Anschlußstück 52, das ein Innengewinde 53 zum Aufschrauben auf die Kupplungselemente 48 des Ringflansches 47 des Katheteransatzes 41 aufweist. Ein koaxialer Außenkonus 54, der in den Innenkonus 43 des Katheteransatzes 41 paßt, wird von einem rohrförmigen Offenhalter 55 nach außen fortgesetzt. Der Offenhalter 55 mit sehr geringen Abmessungen durchdringt im Konnektierungszustand der Teile 41 und 52 den Kreuzschlitz 51 der Elastomerscheibe 50 und hält den Ventilkörper 50 zum Durchströmen einer Flüssigkeit offen. Durch Umschalten des Dreiwegehahns 57 werden die Ein- bzw. Ausgänge B oder C geöffnet bzw. geschlossen.

## Patentansprüche

1. Katheterisierungsbesteck für die Katheterverlegung in einem Blutgefäß, bestehend aus einer Punktionskanüle (10) mit einem Kanülenansatz (16),
einem die Punktionskanüle (10) umgebenden Katheter (13) mit einem Katheteransatz (41), und
einer an dem Kanülenansatz (16) vorgesehenen Griffvorrichtung (25), die an einem Arm (26), der ausgehend vom Kanülenansatz (16) parallel zur Punktionskanüle (10) verläuft, ein mit Abstand neben der Punktionskanüle (10) angeordnetes Griffstück (32) aufweist,
**dadurch gekennzeichnet,**
daß das Griffstück (32) an dem den Katheteransatz (41) überragenden Teil des Armes (26) angeordnet ist, und daß ferner ein Führungsdraht (12) vorgesehen ist, der in dem Lumen der Punktionskanüle (10) axial verschiebbar ist.

2. Katheterisierungsbesteck nach Anspruch 1, dadurch gekennzeichnet, daß das Griffstück (32) zwei zueinander parallele Griffplatten (30, 31) aufweist, deren Ebenen zur Längsachse des Bestecks quergerichtet sind.

3. Punktionsinstrument, bestehend aus einer Stahlnadel (10), die an einem Ende einen Ansatz (16) trägt, welcher eine Griffvorrichtung (25) mit einem Griffstück (32) aufweist, das an einem von dem Ansatz (16) ausgehenden, parallel zur Stahlnadel (10) verlaufenden Arm (26) mit Abstand neben der Stahlnadel (10) angeordnet ist,
**dadurch gekennzeichnet,**
daß das Griffstück (32) zwei zueinander parallele Griffplatten (30,31) aufweist, deren Ebenen zur Längsachse der Stahlnadel (10) quergerichtet sind.

## Claims

1. A catheterization set for placing a catheter in a blood vessel, comprising
a puncture needle (10) provided with a needle hub (16),
a catheter (13) surrounding the puncture needle (10) and having a catheter hub (41) arranged thereon, and
a grip device (25) provided on the needle hub (16) and comprising an arm (26) which, starting from the needle hub (16), extends in parallel along the puncture needle (10) and comprises a grip portion (32) arranged in spaced relationship beside the puncture needle (10),
**characterized in**
that the grip portion (32) is arranged on the part of the arm (26) extending beyond the catheter hub (41), and that a guide wire (12) is provided to be axially displaceable in the lumen of the puncture needle (10).

2. The catheterization set according to claim 1, characterized in that the grip portion (32) comprises two grip plates (30,31) arranged in parallel to each other, the planes of said grip plates (30,31) extending transversely to the longitudinal axis of the set.

3. A puncture instrument, comprising a steel needle (10) carrying on one end thereof a hub (16) comprising a grip device (25) with a grip portion (32), the grip portion (32) being arranged in spaced relationship beside the steel needle (10) on an arm (26) starting from the hub (16) and extending in parallel along the steel needle (10),
**characterized in**
that the grip portion (32) comprises two grip plates (30,31) arranged in parallel to each other, the planes of said grip plates (30,31) extending transversely to the longitudinal axis of the steel needle (10).

## Revendications

1. Equipement de cathétérisme pour la mise en place d'un cathéter dans un vaisseau sanguin, constitué par une canule de ponction (10) comportant un embout (16),
un cathéter (13) qui entoure la canule de ponction (10) et comporte un embout (41), et
un dispositif de préhension (25) qui est prévu sur l'embout (16) de la canule et qui comporte, sur un bras (26) qui s'étend à partir de l'embout (16) de la canule parallèlement à la canule de ponction (10), un élément de préhension (32) disposé à côté de la canule de ponction (10), en en étant espacé,
caractérisé en ce que
l'élément de préhension (32) est disposé sur la partie du bras (26), qui fait saillie au-delà de l'embout (41) du cathéter, et en ce qu'il est prévu en outre un fil de guidage (12), qui peut être déplacé axialement dans la lumière de la canule de ponction (10).

2. Equipement de cathétérisme selon la revendication 1, caractérisé en ce que l'élément de préhension (32) comporte deux plaques de préhension parallèles (30,31), dont les plans sont orientés transversalement à l'axe longitudinal de l'équipement.

3. Instrument de ponction, constitué par une aiguille en acier (10), qui porte à une extrémité un embout (16), lequel possède un dispositif de préhension (25) comportant un élément de préhension (32) qui est disposé à côté de l'aiguille en acier (10) en en étant espacé, sur un bras (26) qui s'étend à partir de l'embout (16), parallèlement à l'aiguille en acier (10),
caractérisé en ce que
l'élément de préhension (32) comporte deux plaques de préhension parallèles (30,31), dont les plans sont orientés transversalement à l'axe longitudinal de l'aiguille en acier (10).
